# EUROPEAN PATENT APPLICATION

(11) **EP 3 430 897 A1**
(43) Date of publication of application: **23.01.2019**
(21) Application number: 16894322.3
(22) Date of filing: 14.03.2016
(51) Int. Cl.: A01K 67/00, A01K 11/00, A01K 21/00, A01K 67/02

(54) **MONITORING DEVICE, MONITORING METHOD, AND MONITORING PROGRAM**

(71) Applicant: Fujitsu Limited, Kawasaki-shi, Kanagawa 211-8588 (JP)
(72) Inventor: HARADA, Takuya, Kawasaki-shi Kanagawa 211-8588 (JP); SHIGENO, Shinji, Fukuoka-shi Fukuoka 812-0007 (JP); WATANABE, Hiroya, Fukuoka-shi Fukuoka 812-0007 (JP); KODAMA, Kenji, Fukuoka-shi Fukuoka 812-0007 (JP); KANAMORI, Akihito, Fukuoka-shi Fukuoka 812-0007 (JP)
(74) Representative: Hoffmann Eitle
(86) International application number: PCT/JP2016/058029
(87) International publication number: WO 2017/158698

(57) **Abstract**

A monitoring device (100) obtains a plurality of captured images including an object, and calculates a movement amount of the object on the basis of a difference between the obtained plurality of captured images. The monitoring device (100) provides notification relating to a sign of calving of the object depending on the movement amount of the object within a predetermined time width.

## Description

### Field

The present invention relates to a monitoring device and the like.

### Background

Dairy farmers farm dairy cows and milk them, for example, as well as assisting calving of breeding cows. Here, since the risk of stillbirth or death of a mother cow increases in case the calving assistance to breeding cows is not properly provided, a rancher periodically makes the rounds and periodically refers to videos from a monitoring camera, thereby determining whether a cow shows a sign of calving.

As described above, in the method of periodically making the rounds and periodically referring to a monitoring camera, a rancher may bear a great burden thereof, whereby it is preferable to automatically informing a rancher of whether a cow shows a sign of calving. With respect to this demand, for example, there is a conventional technique in which acceleration sensors are attached to legs and tails of livestock, tail raising behavior, walking, and the like of livestock are measured, and information associated with livestock calving is output on the basis of the measurement results.

### Citation List

### Patent Literature

Patent Literature 1: Japanese Laid-open Patent Publication No. 2011-234668

### Summary

### Technical Problem

However, with the above-described conventional technique, there may be a problem that a sign of calving is difficult to determine on the basis of a captured image.

For example, in the conventional technique, attaching an acceleration sensor to a tail and a leg of each livestock is a precondition, whereby the technique has not necessarily been an easy-to-use technique for a rancher.

In one aspect, it is an object of the present invention to provide a monitoring device, a method for monitoring, and a monitoring program capable of determining a sign of calving on the basis of a captured image. Solution to Problem

According to an aspect of the embodiment of the invention, a monitoring device includes a control unit that obtains a plurality of captured images including an object, calculates a movement amount of the object on the basis of a difference between the obtained plurality of captured images, and provides a notification relating to a sign of calving of the object depending on the movement amount of the object within a predetermined time width.

### Advantageous Effects of Invention

A sign of calving can be determined on the basis of a captured image.

### Brief Description of Drawings

FIG. 1 is a diagram illustrating a configuration of a system according to the present embodiment.
FIG. 2 is a diagram (1) illustrating an example of processing of a monitoring device.
FIG. 3 is a diagram (2) illustrating another example of the processing of the monitoring device.
FIG. 4 is a block diagram illustrating a configuration of the monitoring device according to the present embodiment.
FIG. 5 is a diagram illustrating an exemplary data structure of a movement amount table.
FIG. 6 is a diagram illustrating an example of mask processing.
FIG. 7 is a flowchart (1) illustrating a processing procedure of the monitoring device according to the present embodiment.
FIG. 8 is a flowchart (2) illustrating another processing procedure of the monitoring device according to the present embodiment.
FIG. 9 is a diagram illustrating an exemplary hardware configuration for executing a monitoring program. Description of Embodiments

Hereinafter, an embodiment of a monitoring device, a method for monitoring, and a monitoring program according to the present invention will be described in detail with reference to the accompanying drawings. Note that this invention is not limited by this embodiment.

### Embodiment

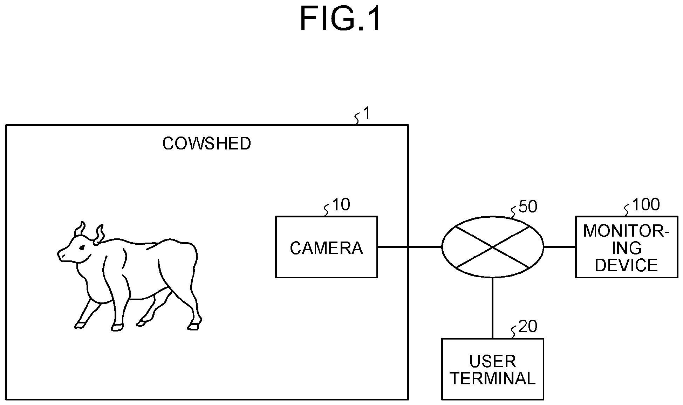

FIG. 1 is a diagram illustrating a configuration of a system according to the present embodiment. As illustrated in FIG. 1, this system includes a camera 10, a user terminal 20, and a monitoring device 100. The camera 10, the user terminal 20, and the monitoring device 100 are mutually connected via a network 50.

The camera 10, which is set in a cowshed 1 in a ranch, for example, is a device that captures a video image included in an imaging range. The camera 10 transmits information associated with a captured video image to the monitoring device 100 via the network 50. In the following descriptions, the information associated with the captured video image is referred to as video information as appropriate. The video information is continuous information of a plurality of pieces of image information.

The user terminal 20 is a terminal used by a rancher or the like in a ranch. The user terminal 20 corresponds to, for example, a mobile phone, a smartphone, and a tablet terminal. When a notification indicating that a sign of calving has been detected is received from the monitoring device 100 to be described later, the user terminal 20 causes a display or the like to display the received information. For example, the user terminal 20 receives a notification indicating that calving information has been detected by e-mail.

The monitoring device 100 is a device that determines whether a cow shows a sign of calving on the basis of the video information received from the camera 10. When it is determined that a sign of calving is shown, the monitoring device 100 notifies the user terminal 20 of the detection of the sign of calving by e-mail or the like.

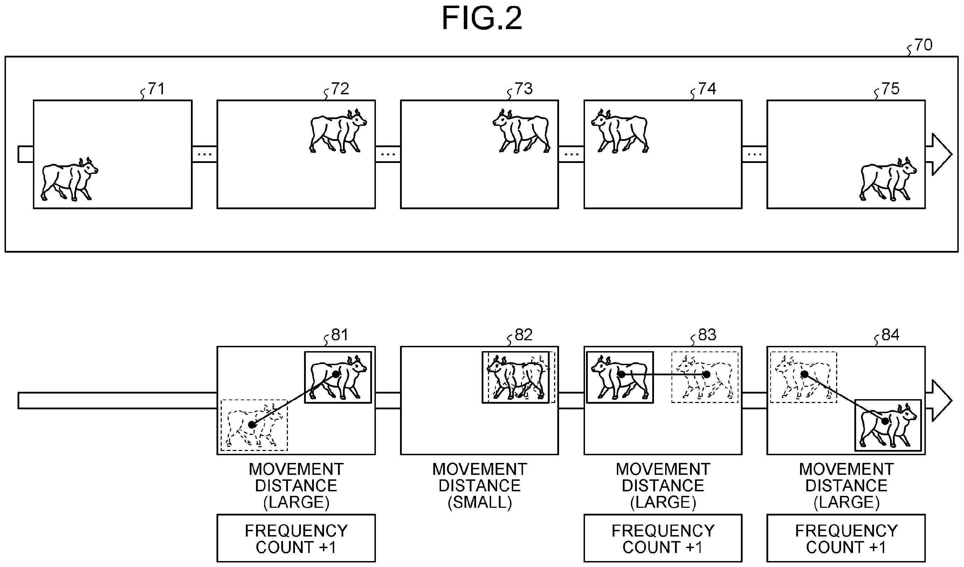

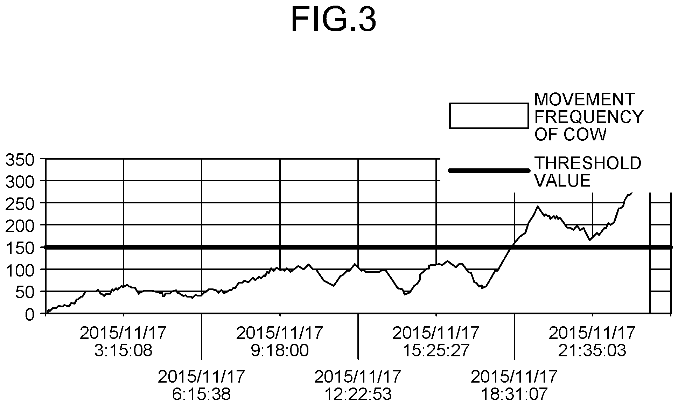

FIGS. 2 and 3 are diagrams illustrating an example of processing of the monitoring device. As illustrated in FIG. 2, the monitoring device 100 extracts image information 71, 72, 73, 74, and 75 from video information 70 at a predetermined time interval tw, and obtains a difference between the extracted previous and next image information 71 to 75, thereby generating difference images 81, 82, 83, and 84. For example, the predetermined time interval tw is set to five seconds.

The difference image 81 is a difference image between the image information 71 and the image information 72. The difference image 82 is a difference image between the image information 72 and the image information 73. The difference image 83 is a difference image between the image information 73 and the image information 74. The difference image 84 is a difference image between the image information 74 and the image information 75.

The monitoring device 100 calculates a movement amount on the basis of the difference image for each difference image, and determines, for each difference image, whether the movement amount is equal to or more than a predetermined movement amount. In the example illustrated in FIG. 2, movement amounts calculated from the difference images 81, 83, and 84 are equal to or more than the predetermined movement amount, and a movement amount calculated from the difference image 82 is less than the predetermined movement amount. The monitoring device 100 increments a frequency count value by "1" for each difference image in which the movement amount is equal to or more than the predetermined movement amount.

The monitoring device 100 repeatedly executes the processing described with reference to FIG. 2, and determines that a sign of calving is shown when a frequency count within a predetermined time width T is equal to or more than a threshold value α. For example, in the present embodiment, the predetermined time width T is set to two hours, and the threshold value α is set to 150. In the example illustrated in FIG. 3, the monitoring device 100 starts the processing on November 17, 2015 at midnight, and the frequency count value per two hours exceeds "150" on November 17, 2015 at 18:31:07. Accordingly, the monitoring device 100 determines that a sign of calving is shown and notifies the user terminal 20 on November 17, 2015 at 18:31:07.

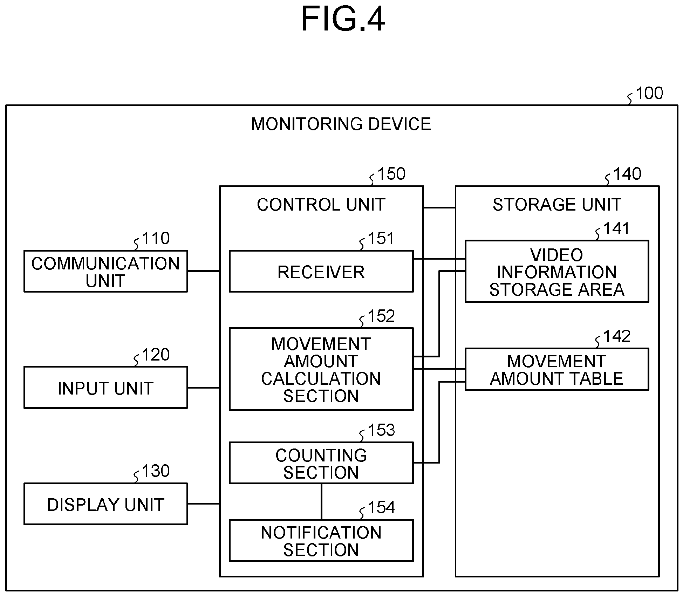

Next, a configuration of the monitoring device 100 will be described. FIG. 4 is a block diagram illustrating a configuration of the monitoring device according to the present embodiment. As illustrated in FIG. 4, the monitoring device 100 includes a communication unit 110, an input unit 120, a display unit 130, a storage unit 140, and a control unit 150.

The communication unit 110 is a processing unit that executes data communication with the camera 10 and the user terminal 20 via the network 50. The communication unit 110 corresponds to a communication device. The control unit 150 to be described later receives video information from the camera 10 via the communication unit 110. The control unit 150 sends a mail to the user terminal 20 via the communication unit 110.

The input unit 120 is an input device for inputting various kinds of information to the monitoring device 100. The input unit 120 corresponds to, for example, a keyboard, a mouse, and a touch panel.

The display unit 130 is a display device that displays various kinds of information output from the control unit 150. The display unit 130 corresponds to a display or a touch panel.

The storage unit 140 includes a video information storage area 141, and a movement amount table 142. The storage unit 140 corresponds to, for example, a semiconductor memory element such as a random access memory (RAM), a read only memory (ROM), and a flash memory, or a storage device such as a hard disk, and an optical disk.

The video information storage area 141 is an area for storing the video information 70 received from the camera 10. For example, the video information 70 is a plurality of pieces of image information arranged in time series, which includes an image of a cow.

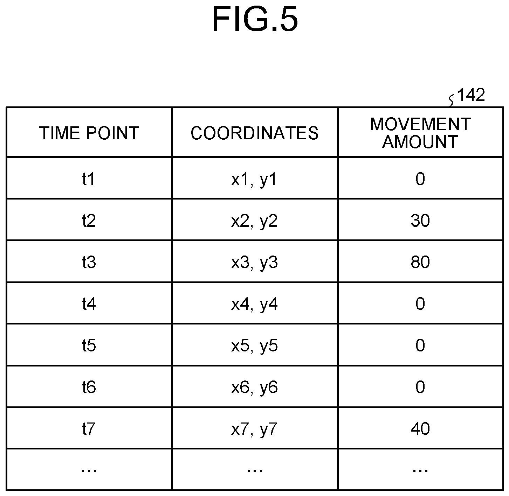

The movement amount table 142 is a table that holds information associated with a movement amount of a cow at each time point. FIG. 5 is a diagram illustrating an exemplary data structure of the movement amount table. As illustrated in FIG. 5, the movement amount table 142 associates a time point, coordinates, and a movement amount with each other.

The control unit 150 includes a receiver 151, a movement amount calculation section 152, a counting section 153, and a notification section 154. The control unit 150 corresponds to, for example, an integrated device such as an application specific integrated circuit (ASIC), and a field programmable gate array (FPGA). Further, the control unit 150 corresponds to, for example, an electronic circuit such as a CPU, and a micro processing unit (MPU).

The receiver 151 is a processing unit that receives the video information 70 from the camera 10. The receiver 151 stores the video information 70 in the storage unit 140.

The movement amount calculation section 152 is a processing unit that obtains the video information 70 stored in the video information storage area 141 and calculates a movement amount of a cow on the basis of a difference between the plurality of pieces of image information included in the video information 70. The movement amount calculation section 152 stores a time point and a movement amount in association with each other in the movement amount table 142. An example of processing of the movement amount calculation section 152 will be described below.

The movement amount calculation section 152 obtains the latest image information, and obtains image information in a frame one frame before. For example, the movement amount calculation section 152 obtains image information from the video information 70 at the predetermined time interval tw. Therefore, the image information in the frame one frame before is the image information at a time point tw time prior to the time point of the latest image information. The image information in the frame one frame before is stored in a predetermined area in the storage unit 140 after being subject to mask processing to be described later.

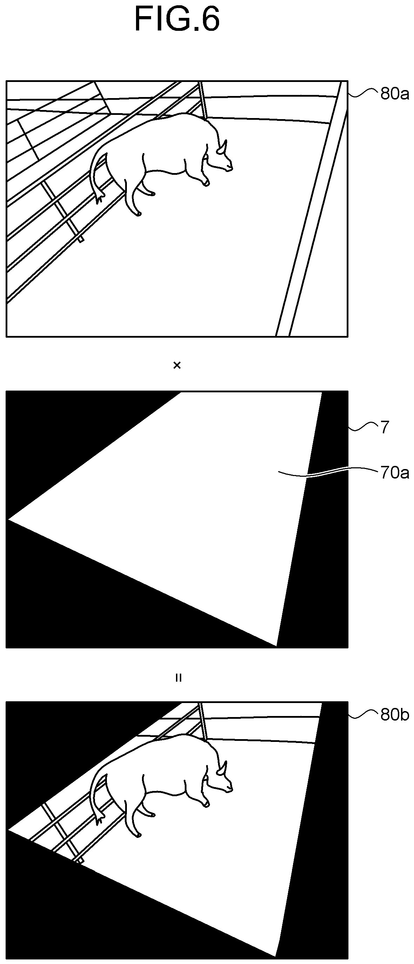

The movement amount calculation section 152 executes the mask processing on the latest image information, whereby a pixel value of an unnecessary area in the entire area of the image information is converted into a predetermined pixel value. FIG. 6 is a diagram illustrating an example of the mask processing. In the example illustrated in FIG. 6, the latest image information is referred to as image information 80a. The movement amount calculation section 152 superimposes the image information 80a and mask information 7, and executes processing of converting a pixel value of the area other than the area overlapping a mask area 70a in the image information 80a into a predetermined pixel value, thereby generating image information 80b. The movement amount calculation section 152 stores the latest image information 80b having been subject to the mask processing in a predetermined area of the storage unit 140 as image information in the frame one frame before.

The movement amount calculation section 152 executes a grayscale conversion on the latest image information and the image information in the frame one frame before. The movement amount calculation section 152 obtains a difference between the latest image information and the image information in the frame one frame before having been subject to the grayscale conversion, thereby generating a difference image. Further, the movement amount calculation section 152 generates a binary image obtained by binarizing the difference image. For example, the movement amount calculation section 152 compares a pixel value of each pixel of the difference image with a predetermined value, sets a pixel having a pixel value equal to or more than the predetermined value to "1", and sets a pixel having a pixel value less than the predetermined value to "0".

The movement amount calculation section 152 detects a cow on the basis of the binary image. For example, the movement amount detection section 152 scans the binary image and connects the areas in which the pixel value "1" continues, thereby identifying a target area. The movement amount calculation section 152 compares the target area with cow area information corresponding to a size of a cow, and detects the target area as a cow area when an absolute value of a difference between the size of the target area and the size of the cow area is less than a predetermined threshold value.

Movement amount calculation section 152 compares coordinates of the gravity center of the target area with a previous cow coordinates, and calculates movement amounts in an X direction and a Y direction. When the movement amount in the Y direction is 25 pixels or more, the movement amount calculation section 152 stores the time point, the coordinates, and the movement amount in association with each other in the movement amount table 142. The time point corresponds to a time point at which the latest image information is captured. The coordinates correspond to coordinates of the gravity center of the target area determined as a cow area. The movement amount corresponds to a distance between, in the movement amount table 142, coordinates at the previous time point and coordinates at the current time point. Here, the movement amount calculation section 152 may store only the movement amount in the Y direction in the movement amount table 142.

On the other hand, when the movement amount in the Y direction is less than 25 pixels, the movement amount calculation section 152 stores the time point, the coordinates, and the movement amount "0" in association with each other in the movement amount table 142. Here, the movement amount calculation section 152 sets the coordinates to the coordinates of the frame one frame before.

The counting section 153 is a processing unit that counts the frequency count value on the basis of the movement amount table 142. For example, the counting section 153 identifies records between the current time point and the time point two hours before the current time point in the movement amount table 142, and counts the records in which the movement amount is not "0" as the frequency count value. When the frequency count value exceeds "150" for the first time, the counting section 153 determines that the cow shows a sign of calving, and outputs the determination result to the notification section 154.

The notification section 154 is a processing unit that notifies, when the counting section 153 determines that the cow shows a sign of calving, the user terminal 20 of information indicating that the cow shows the sign of calving by e-mail or the like.

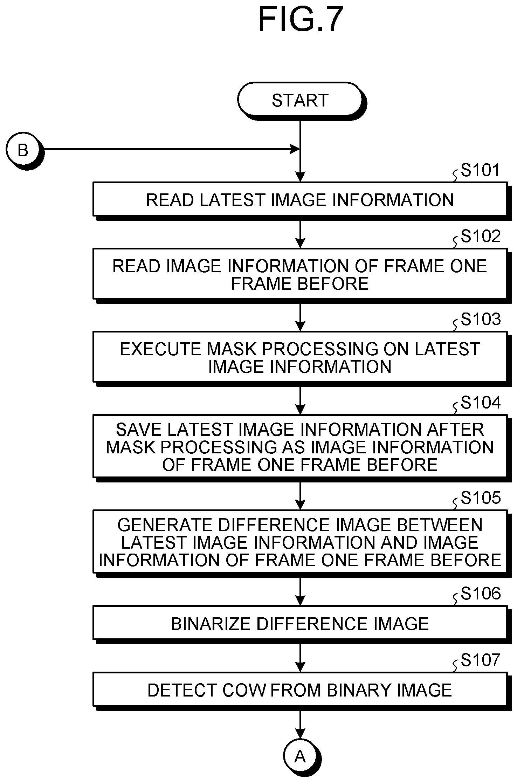

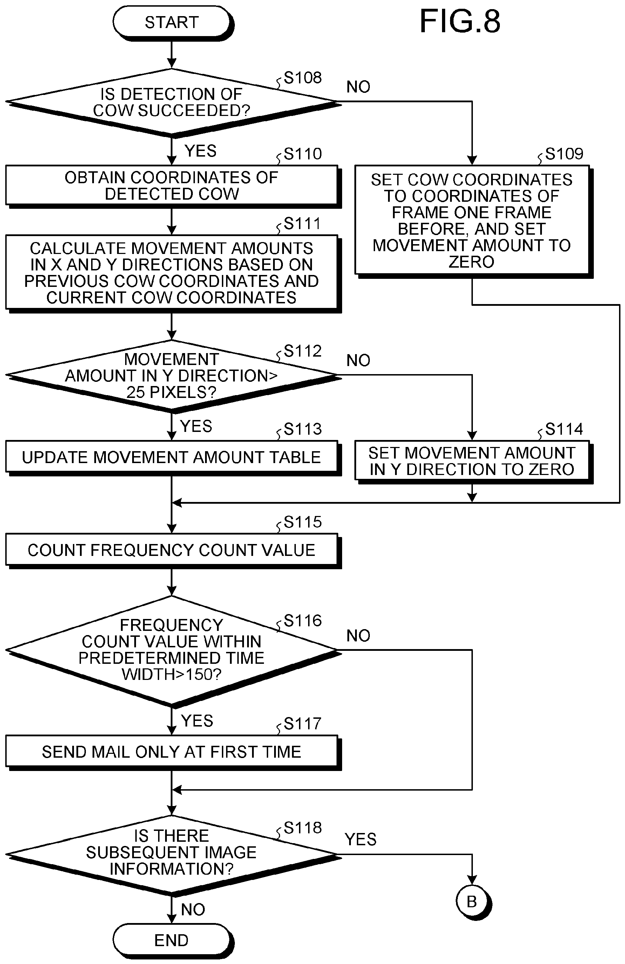

Next, an exemplary processing procedure of the monitoring device 100 according to the present embodiment will be described. FIGS. 7 and 8 are flowcharts illustrating the processing procedure of the monitoring device according to the present embodiment. As illustrated in FIG. 7, the movement amount calculation section 152 of the monitoring device 100 reads the latest image information (step S101), and reads the image information in the frame one frame before (step S102).

The movement amount calculation section 152 executes the mask processing on the latest image information (step S103). The movement amount calculation section 152 saves the latest image information having been subject to the mask processing as the image information in the frame one frame before (step S104).

The movement amount calculation section 152 generates a difference image between the latest image information and the image information in the frame one frame before (step S105). The movement amount calculation section 152 binarizes the difference image (step S106), detects a cow from the binary image (step S107), and moves to step S108 in FIG. 8.

Next, FIG. 8 will be described. The movement amount calculation section 152 determines whether the detection of a cow is succeeded (step S108). When the detection of a cow is not succeeded (step S108, No), the movement amount calculation section 152 sets the cow coordinates to the coordinates of the frame one frame before, sets the movement amount to zero (step S109), and moves to step S115.

When the detection of a cow is succeeded (step S108, Yes), the movement amount calculation section 152 obtains the coordinates of the detected cow (step S110). The movement amount calculation section 152 calculates the movement amounts in the X and Y directions on the basis of the previous cow coordinates and the current cow coordinates (step S111).

The movement amount calculation section 152 determines whether the movement amount in the Y direction exceeds 25 pixels (step S112). When the movement amount in the Y direction exceeds 25 pixels (step S112, Yes), the movement amount calculation section 152 registers the time point, the coordinates, and the movement amount in the Y direction in the movement amount table 142 so that the movement amount table 142 is updated (step S113), and moves to step S115.

When the movement amount in the Y direction is equal to or less than 25 pixels (step S112, No), the movement amount calculation section 152 sets the movement amount in the Y direction to zero (step S114), and moves to step S115.

The counting section 153 counts the frequency count value on the basis of the movement amount table 142 (step S115). The counting section 153 determines whether the frequency count value within a predetermined time width exceeds 150 (step S116). When the frequency count value within the predetermined time width is equal to or less than 150 (step S116, No), the counting section 153 proceeds to step S118.

On the other hand, when the frequency count value within the predetermined time width exceeds 150 (step S116, Yes), the counting section 153 proceeds to step S117. The notification section 154 of the monitoring device 100 sends a mail to the user terminal 20 only at the first time (step S117). When there is a subsequent image information (step S118, Yes), the monitoring device 100 proceeds to step S101 in FIG. 7. When there is no subsequent image information (step S118, No), the monitoring device 100 terminates the process.

Next, effects of the monitoring device 100 according to the present embodiment will be described. The monitoring device 100 obtains a plurality of pieces of image information including a cow, calculates the movement amount of the cow on the basis of the difference between the plurality of pieces of image information, and provides a notification relating to the sign of calving of the cow depending on the movement amount of the cow within the predetermined time width. As described above, with the use of the monitoring device 100, the sign of calving of the cow can be determined using only the video information obtained from the camera 10.

The monitoring device 100 repeatedly executes the processing of detecting a position of the cow by calculating a difference between the image information captured at different time points, and calculates the movement amount of the object on the basis of the previous position of the cow and the currently calculated position of the cow. Therefore, the movement amount of the cow can be appropriately calculated using a simple method.

The monitoring device 100 stores the information regarding whether the movement amount is equal to or more than the predetermined movement amount and the time point in association with each other in the storage unit 140, counts the number of times at which the movement amount becomes equal to or more than the predetermined movement amount within the predetermined time width, and provides the notification indicating that the object shows a sign of calving when the number of times is equal to or more than a threshold value. Accordingly, even when there is an error in the movement amount at each time point, an influence of the error can be suppressed by a threshold comparison, whereby it is possible to accurately determine whether the cow shows a sign of calving.

Meanwhile, the monitoring device 100 may determine that the cow shows a sign of calving by further executing other processing. Hereinafter, other processing (1) and (2) executed by the monitoring device 100 will be described.

Other processing (1) executed by the monitoring device 100 will be described. Although the movement amount calculation section 152 of the monitoring device 100 calculates the movement amounts in the X direction and the Y direction, and stores the movement amount in the Y direction in the movement amount table 142 when the movement amount in the Y direction is equal to or more than 25 pixels according to the descriptions above, it is not limited thereto. The movement amount calculation section 152 stores, not only the movement amount in the Y direction, but also a distance between the previous cow coordinates and the coordinates of the gravity center of the target area determined as a cow in the movement amount table 142.

Here, the counting section 153 calculates a total amount of the movement amounts between the current time point and the time point two hours before the current time point on the basis of the movement amount table 142. The counting section 153 determines that the cow shows a sign of calving when the total amount of the movement amounts between the current time point and the time point two hours before the current time point is equal to or more than a predetermined value.

As described above, in other processing (1), the monitoring device 100 calculates the total amount of the movement amounts within the predetermined time width, and determines that the cow shows a sign of calving when the total amount becomes equal to or more than the predetermined value, whereby the sign of calving of the cow can be determined using only the video information obtained from the camera 10.

Other processing (2) executed by the monitoring device 100 will be described. Although the monitoring device 100 determines whether the cow shows a sign of calving by the threshold comparison of the frequency count value within the predetermined time width in the descriptions above, it is not limited thereto. For example, the monitoring device 100 may calculate a total period of time of the period of time in which a direction of a cow's tail faces upward, and determines that the cow shows a sign of calving when the total period of time exceeds five minutes.

The movement amount calculation section 152 detects the cow in a similar manner to the processing above, and executes a template matching or the like when the detection of the cow is succeeded, thereby identifying an area of the cow's tail. The movement amount calculation section 152 determines whether the area of the cow's tail faces upward, and stores the determination result in the movement amount table 142 or the like. Note that the upward direction of the cow in the image information is set in the movement amount calculation section 152 in advance.

When the frequency count value within the predetermined time width exceeds 150, or when the total value of the period of time in which the direction of the cow's tail faces upward exceeds "5 minutes", the counting section 153 determines that the cow shows a sign of calving.

Alternatively, when the frequency count value within the predetermined time width exceeds 150 and the total value of the period of time in which the direction of the cow's tail faces upward exceeds "5 minutes", the counting section 153 determines that the cow shows a sign of calving.

The monitoring device 100 determines whether the sign of calving is shown by focusing on the direction of the cow's tail in addition to the movement amount, whereby the sign of calving can be determined more accurately.

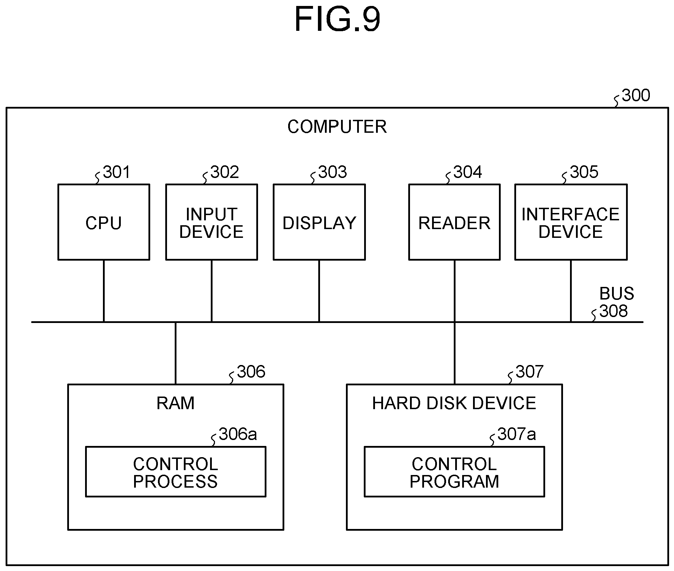

Next, an exemplary hardware configuration for executing a monitoring program that exerts a function similar to the monitoring device 100 described in the above embodiment will be described. FIG. 9 is a diagram illustrating the exemplary hardware configuration for executing the monitoring program.

As illustrated in FIG. 9, a computer 300 includes a CPU 301 that executes various arithmetic processing, an input device 302 that accepts input of data from a user, and a display 303. Further, the computer 300 includes a reader 304 that reads a program or the like from a storage medium, and an interface device 305 that exchanges data with another computer via a network. Furthermore, the computer 300 includes a RAM 306 that temporarily stores various information, and a hard disk device 307. Each of the devices 301 to 307 is connected to a bus 308.

The hard disk device 307 includes a control program 307a. The CPU 301 reads the control program 307a and expands the control program 307a in the RAM 306. The control program 307a functions as a control process 306a. For example, the processing of the control process 506a corresponds to the processing of the control unit 150.

Here, the control program 307a may not necessarily be stored in the hard disk device 307 from the beginning. For example, each program is stored in a "portable physical medium" to be inserted into the computer 300 such as a flexible disk (FD), a CD-ROM, a DVD disk, a magnetooptic disk, and an IC card. Then, the computer 300 may read and execute the control program 307a.

### Reference Signs List

- 100: monitoring device
- 110: communication unit
- 120: Input unit
- 130: display unit
- 140: storage unit
- 141: video information storage area
- 142: movement amount table
- 150: control unit
- 151: receiver
- 152: movement amount calculation section
- 154: notification section

## Claims

1. A monitoring device, comprising:
a control unit that obtains a plurality of captured images including an object, calculates a movement amount of the object on the basis of a difference between the obtained plurality of captured images, and provides a notification relating to a sign of calving of the object depending on the movement amount of the object within a predetermined time width.

2. The monitoring device according to claim 1, wherein the control unit detects a position of the object by calculating a difference between captured images captured at different time points, and calculates the movement amount of the object on the basis of positions of the object continuously detected in time series.

3. The monitoring device according to claim 1 or 2, wherein the control unit stores information regarding whether the movement amount is equal to or more than a predetermined movement amount and a time point in association with each other in a storage unit, counts the number of times at which the movement amount becomes equal to or more than the predetermined movement amount within a certain time width, and provides a notification indicating that the object shows the sign of calving when the counted number of times is equal to or more than a threshold value.

4. The monitoring device according to claim 1 or 2, wherein the control unit calculates a total amount of the movement amounts by adding the movement amounts, and provides the notification indicating that the object shows the sign of calving when the total amount of the movement amounts is equal to or more than a predetermined value within a certain time width.

5. A monitoring device, comprising:
a control unit that analyzes a captured image in a plurality of frames within a predetermined period of time, and executes predetermined notification control depending on a movement amount of an image portion having a characteristic corresponding to a cow between frames and a direction of a tail of the cow extracted from the captured image.

6. The monitoring device according to claim 5, wherein the control unit provides a predetermined notification when the movement amount is equal to or more than a predetermined movement amount or when a total value of a period of time in which the direction of the tail of the cow is in the direction same as a preset direction becomes equal to or more than a threshold value.

7. A method for monitoring, the method in which a computer executes a process comprising:
obtaining a plurality of captured images including an object;
calculating a movement amount of the object on the basis of a difference between the obtained plurality of captured images; and
providing a notification relating to a sign of calving of the object depending on the movement amount of the object within a predetermined time width.

8. The method for monitoring according to claim 7, wherein in the calculating process, a position of the object is detected by calculating a difference between captured images captured at different time points, and the movement amount of the object is calculated on the basis of positions of the object continuously detected in time series.

9. The method for monitoring according to claim 7 or 8, wherein information regarding whether the movement amount is equal to or more than a predetermined movement amount and a time point in association with each other are stored in a storage unit, the number of times at which the movement amount becomes equal to or more than the predetermined movement amount within a certain time width is counted, and a notification indicating that the object shows the sign of calving is provided when the counted number of times is equal to or more than a threshold value.

10. The method for monitoring according to claim 7 or 8, wherein a total amount of the movement amounts is calculated by adding the movement amounts, and the notification indicating that the object shows the sign of calving is provided when the total amount of the movement amounts is equal to or more than a predetermined value within a certain time width.

11. A method for monitoring, the method in which a computer executes a process comprising:
analyzing a captured image in a plurality of frames within a predetermined period of time; and
executing predetermined notification control depending on a movement amount of an image portion having a characteristic corresponding to a cow between frames and a direction of a tail of the cow extracted from the captured image.

12. The method for monitoring according to claim 11, wherein in the process of executing notification control, a predetermined notification is provided when the movement amount is equal to or more than a predetermined movement amount or when a total value of a period of time in which the direction of the tail of the cow is in the direction same as a preset direction becomes equal to or more than a threshold value.

13. A monitoring program that causes a computer to execute a process comprising:
obtaining a plurality of captured images including an object;
calculating a movement amount of the object on the basis of a difference between the obtained plurality of captured images; and
providing a notification relating to a sign of calving of the object depending on the movement amount of the object within a predetermined time width.

14. The monitoring program according to claim 13, wherein in the calculating process, a position of the object is detected by calculating a difference between captured images captured at different time points, and the movement amount of the object is calculated on the basis of positions of the object continuously detected in time series.

15. The monitoring program according to claim 13 or 14, wherein information regarding whether the movement amount is equal to or more than a predetermined movement amount and a time point in association with each other are stored in a storage unit, the number of times at which the movement amount becomes equal to or more than the predetermined movement amount within a certain time width is counted, and a notification indicating that the object shows the sign of calving is provided when the counted number of times is equal to or more than a threshold value.

16. The monitoring program according to claim 13 or 14, wherein a total amount of the movement amounts is calculated by adding the movement amounts, and the notification indicating that the object shows the sign of calving is provided when the total amount of the movement amounts is equal to or more than a predetermined value within a certain time width.

17. A monitoring program that causes a computer to execute a process comprising:
analyzing a captured image in a plurality of frames within a predetermined period of time; and
executing predetermined notification control depending on a movement amount of an image portion having a characteristic corresponding to a cow between frames and a direction of a tail of the cow extracted from the captured image.

18. The monitoring program according to claim 17, wherein in the process of executing notification control, a predetermined notification is provided when the movement amount is equal to or more than a predetermined movement amount or when a total value of a period of time in which the direction of the tail of the cow is in the direction same as a preset direction becomes equal to or more than a threshold value.
